# EUROPEAN PATENT SPECIFICATION

(11) **EP 3 199 079 B1**
(45) Date of publication and mention of the grant of the patent: **04.03.2020**
(21) Application number: 15729324.2
(22) Date of filing: 04.01.2015
(51) Int. Cl.: A47K 7/04, A61B 17/54, A61H 9/00

(54) **BODY CLEANING HEAD AND BODY CLEANING TOOL**
KÖRPERREINIGUNGSKOPF UND KÖRPERREINIGUNGSWERKZEUG
TÊTE DE NETTOYAGE POUR LE CORPS ET OUTIL DE NETTOYAGE POUR LE CORPS

(30) Priority: 22.09.2014 CN 201410486475
(43) Date of publication of application: 02.08.2017
(73) Proprietor: BOE Technology Group Co., Ltd., Beijing 100015 (CN)
(72) Inventor: LI, Liang, Beijing 100176 (CN)
(74) Representative: Brötz, Helmut
(86) International application number: PCT/CN2015/070019
(87) International publication number: WO 2016/045237

(56) References cited:
- CN-A- 102 525 320
- CN-Y- 201 067 367
- DE-C2- 4 237 940
- FR-A5- 2 152 061
- US-A- 4 692 140
- US-A- 6 021 539
- US-A1- 2008 087 853
- US-A1- 2011 082 415

## Description

### TECHNICAL FIELD

Embodiments of the present invention relate to a body cleaning head and a body cleaning tool.

### BACKGROUND

For a person to whom it is inconvenient to clean his/her body in a conventional manner, usually, cleaning is usually carried out by using a body cleaner with a body cleaning head; by contacting the body cleaning head with the person's skin, his/her body can be cleaned and at the same time dirty water after cleaning can be drawn back to the cleaning device.

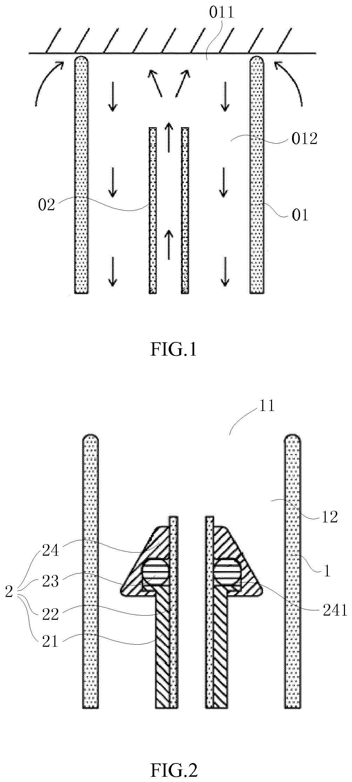

In order to achieve functions of cleaning body and drawing back the dirty water after cleaning at the same time, as illustrated in FIG. 1, the body cleaning head comprises an outer case 01, the outer case 01 is provided therein with a suction port 011 and a return flow passage 012 communicating with the suction port, the outer case 01 is also provided therein with a outflow pipe 02, the clean water flows in a direction as indicated by an arrow in the outflow pipe 02 and is sprayed onto the skin from the outlet of the outflow pipe. After cleaning, dirty water is drawn into the flow return passage 012 by a suction motor (not shown) from the suction port 011 in a direction indicated by an arrow. When the suction motor is operated, it can not be kept at a stable state for a long time.

Therefore, the unstable operation of the suction motor causes the suction effect vary from time to time, when the suction power is large, clean water to be sprayed out of the outlet is drawn away by the suction motor before it reaches the skin, and the effect of cleaning the body cannot be achieved; and when the suction power is small, dirty water after cleaning cannot be effectively drawn back to the body cleaning device in time, but will flow to other positions, the function of dirty water recycle cannot be achieved well.
US 4 692 140 A discloses a lavage/suction tip comprising an elongated barrel with a first pliable conical shield extending outwardly from the distal tip of the barrel and a second substantially rigid conical shield which is slidable upon the barrel. The lavage/suction tip is utilized to provide irrigating fluid to a surgical site while providing suction to remove fluids and debris away from the surgical site. The first shield helps to eliminate splashback of the fluid. The second shield may be positioned to surround the first shield to provide firm support about the first shield, yet with a flexible, pliable tip perimeter. Alternatively, the second shield may be secured on the tip spaced away from the first shield so that the pliable first shield is used alone and may be easily, manually formed to more closely match the tip of the first shield to the working area or surface of the surgical site.
US 2011/082415 A1 discloses a microdermabrasion device for treating skin comprising a handpiece assembly having a distal end and a proximal end. The handpiece assembly includes at least one delivery conduit and at least one waste conduit. The microdermabrasion device additionally comprises a tip configured to be positioned along the distal end of the handpiece assembly, wherein the tip is adapted to contact skin surface. In several embodiments, the tip comprises a lip, a first opening in fluid communication with the fluid delivery conduit and a second opening in fluid communication with the waste conduit. In one embodiment, the device includes one or more abrasive elements positioned along a distal end of the tip, wherein the abrasive elements are configured to selectively remove skin as the tip is moved relative to a skin surface. In some embodiments, the delivery conduit is configured to selectively deliver at least one time-release material to the skin surface being treated. US 2008/087853 A1 discloses a pinch valve having a hose-like valve member with a flexible peripheral wall adjacent to whose outer periphery two diametrally opposite pinch elements are arranged able to be moved toward and away from one another with a greater or lesser pinching of the peripheral wall while performing an operating movement athwart the valve member longitudinal axis. An annular actuating ring coaxially surrounding the valve member may act on the pinch elements for causing the operating movement. The actuating ring is placed in a working space surrounded radially from the outside by a peripheral valve housing wall and is able to be acted on by drive means separate from the actuating ring with a drive force controlling the actuating movement. FR 2 152 061 A5 discloses a valve includes an annular passage for fluid to pass through defined by a flexible wall and an obstructor, and an operating mechanism for bringing about engagement between the wall and the obstructor to close the passage, the operating mechanism comprising first means, e.g. a bellows to produce axial movement and a camming member moved axially by fluid pressure applied to the bellows to produce radial movement of the wall. The camming surface of the camming member acts on ball bearings. The flexible wall may be constituted by a sleeve with a flexible ring mounted between the sleeve and the ball bearings or the ring may be omitted or the sleeve may be omitted in which case the ring constitutes the flexible wall. The bellows may be activated by the pressure of the controlled fluid.
CN 102 525 320 A discloses a human body dry cleaning machine, which comprises a shell. The human body dry cleaning machine is characterized by further comprising a human body brush structure, a lotion delivering device, a dirt recycling device, a power supply device and an electric heating device, the human body brush structure is used for brushing and removing dirt on the surface of a human body, the lotion delivering device is used for delivering lotion to an area of the human body brush structure, the dirt recycling device is used for recycling the dirt brushed and removed by the human body brush structure, the power supply device is used for supplying power for the human body brush structure so as to lead the human body brush structure to rotate at high speed,; and is used for supplying power for the dirt recycling device so that the dirt recycling device generates negative pressure and recycles the dirt, and the electric heating device is used for heating the lotion. The human body dry cleaning machine has the advantages of simple structure and convenience in use, simultaneously, the dirt on the human body can be removed by a small quantity of lotion, and water resources are saved.
CN 201 067 367 Y discloses a pipe nozzle structure which can lead the pipe nozzle to move easily along the body which is attached by excrement and lead the liquid which is sprayed from the interior of the pipe nozzle to the excrement not to splash outwards. The structure of pipe nozzle is that the pipe nozzle is connected with a suction device which is used for removing the excrement attached to the body by suction; the pipe nozzle structure is characterized in that the end at one side of an opening facing to the articles to be sucked is provided with a plurality of front ends which are provided with round bulges along the circumferential direction; when the suction device is in use, the external air is guided into the pipe nozzle through the gap of the bulges.
US 6021539 A discloses a hand-held washing device has a housing, a rotor rotatable in the housing, a washing tool attached to the rotator and rotatable therewith, a holder connected with the housing, and a handle telescopably connected with the holder.
DE 4 237 940 C2 discloses a body-cleaning utensil. The body-cleaning utensil consists of a hand-operated appliance with washer-head and handle. In the centre of the head is an opening for dispensing washing solution and surrounded by suction-holes. The side of the suction-holes facing the dispenser-opening has a seal so as to form a vacuum-chamber sealed from the ambient atmosphere. Next to the suction-holes is an annular washer made of open-cellular, air and liquid-permeable plastics or rubber. The wall of the central area supplying the washing solution is sealed off right up to the person's skin being washed. The body washer prevents water from spurting out, frictions the body, and protects the body from chill after washing.

### SUMMARY

At least one embodiment of the present disclosure provides a body cleaning head and a body cleaning tool, which are capable of achieving a relative stable state of the water outflow velocity from an outlet and that of the suction power of a suction port, and achieving good effect of cleaning the body and drawing dirty water back to a cleaning machine.

At least one embodiment of the present disclosure provides a body cleaning head comprising: an outer case provided with a suction port and a flow return passage communicated with the suction port; and a water pipe assemble provided in the flow return passage and comprising a pipe bracket fixedly provided in the outer case. A water discharge hose is inserted and fitted within the pipe bracket. An elastic body is provided to sleeve the end of the water discharge hose at the end of the pipe bracket and a slide sleeve is provided to sleeve the elastic body and the water discharge hose, both the elastic body and the slide sleeve are configured to surround the water discharge hose, the elastic body is located between the pipe bracket and the slide sleeve, when the body cleaning head sucks air, the slide sleeve axially moves relative to the pipe bracket under an action of the airflow pressure in such a manner that the elastic body is deformed such that an inner diameter of the elastic body is reduced and accordingly, an inner diameter of a portion of the water discharge hose corresponding to the elastic body is varied with the airflow pressure.

According to one embodiment of the present disclosure, the slide sleeve is a conical sleeve, and an end of the slide sleeve with a smaller diameter is provided adjacent the suction port of the outer case.

According to one embodiment of the present disclosure, the inner wall of the slide sleeve is formed with an annular slot, the elastic body is placed in the annular slot, and an end of the pipe bracket is inserted into the annular slot and abuts against the elastic body.

According to one embodiment of the present disclosure, the elastic body is an elastic ring, and the maximum diameter of the annular slot matches the outer diameter of the elastic ring.

According to one embodiment of the present disclosure, the elastic body comprises a plurality of elastic block.

According to one embodiment of the present disclosure, the water outlet of the water discharge hose is located in the outer case, and the suction port of the outer case is provided at a distance from the water outlet of the water discharge hose in the axial direction.

According to one embodiment of the present disclosure, the distance from the suction port of the outer case to the water outlet of the water discharge hose in the axial direction is less than or equal to a spraying distance of the water outlet.

According to one embodiment of the present disclosure, the distance from the suction port of the outer case to the water outlet of the water discharge hose in the axial direction is less than the spraying distance of the water outlet by from 1cm to 2 cm.

According to one embodiment of the present disclosure, the elastic body is made of a rubber material.

At least one embodiment of the present disclosure further provides a body cleaning tool comprising a water supply motor and a suction motor, and further comprising any one of the body cleaning heads as described above. The water supply motor communicates with the water discharge hose, and the suction motor communicates with the return flow passage of the body cleaning head, the suction motor can suck ambient air and the water flow sprayed by the water discharge hose back into the cleaning tool.

### BRIEF DESCRIPTION OF THE DRAWINGS

In order to clearly illustrate the technical solution of the embodiments of the invention, the drawings of the embodiments will be briefly described in the following; it is obvious that the described drawings are only related to some embodiments of the invention and thus are not limitative of the invention.
FIG. 1 is a schematic view showing the structure of the body cleaning head known by the inventor;
FIG. 2 is a schematic sectional view showing the structure of the body cleaning head, which is in a non-open state, according to an embodiment of the present disclosure;
FIG. 3 is a schematic sectional view showing the structure of the body cleaning head, which is in an operation state and contacts a skin, according to the embodiment of the present disclosure; and
FIG. 4 is a schematic sectional view showing the structure of the body cleaning head, which is in an operation state but does not contact the skin, according to the embodiment of the present disclosure.

### DETAILED DESCRIPTION

In order to make objects, technical details and advantages of the embodiments of the invention apparent, the technical solutions of the embodiment will be described in a clearly and fully understandable way in connection with the drawings related to the embodiments of the invention. It is obvious that the described embodiments are just a part but not all of the embodiments of the invention. Based on the described embodiments herein, those skilled in the art can obtain other embodiment(s), without any inventive work, which should be within the scope of the invention.

In the description of the present disclosure, it should be noted that the orientation or position relationship indicated by the terms "center", "up", "down", "front", "rear", "left", "right", "vertical", "horizontal", "top", "bottom", "inner", "outer" and so on is based on the orientation or position relationship as indicated by the figures, and is only intended to facilitate the description of the present disclosure and simplify the description, and should not be construed as to indicate or imply the referred devices or members must have the particular orientation, is constructed and operated by the particular construction, and thus should not be interpreted as limitations to the present disclosure. In addition, the terms "first", "second" and so on are only used for descriptive purpose, and should not be interpreted as indicating or implying the significance or implicitly indicating the number of the indicated technical features. Therefore, the feature prefixed with the terms "first", "second" can clearly or implicitly indicate comprising one or more such features.

In the description of the present disclosure, it should be noted that, unless expressly stated and defined to the contrary, the terms "amount", "connect", "connection" should be interpreted in its broadest sense, for example, it may be a permanent connection or a releasable connection, or an integral connection; it may be a mechanical connection, or a electrical connection; it may be a direct connection or an indirect connection via an intervenient member; it may be communication between the interiors of two members. The meaning of the above terms in the present disclosure can be suitably understood by the person skilled in the art according to the practical situation.

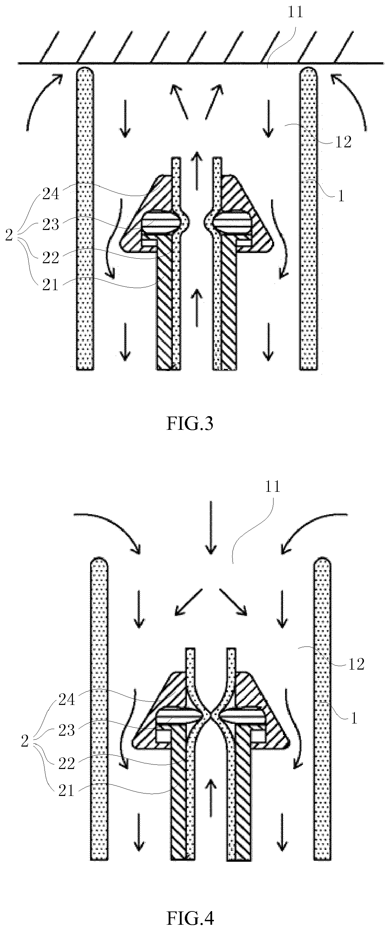

FIG. 2 and FIG. 3 are schematic views showing the sectional structure of a body cleaning head according to one embodiment of the present disclosure. Referring to FIG. 2 and FIG. 3 the body cleaning head according to the present embodiment includes an outer case 1 provided with a suction port 11 and provided therein with a reflow passage 12 communicating with the suction port 11; and a water pipe assembly 2 provided within the reflow passage 12, the water pipe assembly 2 includes a pipe bracket 21 fixedly provided in the outer case 1, a water discharge hose 22 is inserted and fitted within the pipe bracket 21, an end of the water discharge hose 22 adjacent to the suction port 11 is protruded out of the pipe bracket 21, and an elastic body 23 and a slide sleeve 24 are provided around the protruded end, the elastic body 23 is located between the pipe bracket 21 and the slide sleeve 24, and the slide sleeve 24 is axially movable relative to the pipe bracket 21. When the cleaning head sucks air, the air flow in the reflow passage 12 pushes the slide sleeve 24 to move towards the pipe bracket 21 and press the elastic body 23, such that the size of the elastic body 23 in the radial direction of the water discharge hose is enlarged, and thus the water discharge hose 22 is pressed so that inner diameter of the portion of the water discharge hose 22 corresponding to the elastic body 23 is reduced. When the air pressure of the suction port is changed from large to small, the slide sleeve 24 is moved in a direction away from the pipe bracket 21, such that the size of the elastic body 23 in the radial direction of the water discharge hose is reduced, and thus the inner diameter of the portion of the water discharge hose 22 corresponding to the elastic body 23 is increased.

The body cleaning head provided by the present embodiment includes the outer case 1 and the water pipe assembly 2. When the body cleaning head is put in operation, air suction and water discharging are performed at the same time; in this case, referring to FIG. 4, when the cleaning head does not contact with skin yet, the air pressure in the reflow passage 12 is maximum and is equal to the atmospheric pressure, and the air flow in the reflow passage 12 flows along the direction as indicated by the downward arrow in the drawing, and pushes the slide sleeve 24 to move in the direction approaching the pipe bracket 21 towards the maximum extent and presses the elastic body 23 in such a manner that the size of the elastic body 23 in the radial direction of the pipe discharging hose is increased and thus the water discharge hose 22 is pressed to be deformed to the maximum extent and accordingly the inner diameter of the portion of the water discharge hose 22 corresponding to the elastic body is reduced toward the minimum extent, approaching to a close state; therefore, when the cleaning head does not contact with the skin, the water will not be sprayed from the cleaning head. Given the water pressure in the water discharge hose 22 is constant, the spray distance of the water outlet is related to both the cross-sectional area of the water outlet and the pressure in the suction port 11. Therefore, when the pressure of the suction port 11 is not stable, the spray distance of the water outlet can be guaranteed by changing the cross-sectional area of the water outlet. Referring to FIG. 3, when the cleaning head contacts with the skin, a relative large resistant force is generated when air is sucked, but the air pressure in the reflow passage 12 is less than the atmospheric pressure, the pressure acting on the slide sleeve 24 is reduced, and thus the displacement amount, by which the slide sleeve 24 is moved, is reduced. Accordingly, the deformation amount of the elastic body 23 is reduced, the inner diameter of the portion of the water discharge hose 22 corresponding to the elastic body 23 is increased, and the water discharge hose 22 is opened, so that the water in the water discharge hose 23 can be flowed along the direction as indicated by the upward arrow in the drawing and is sprayed on the skin to clean the skin. During cleaning, the suction power may be varied, when the suction power is increased, the pressure in the reflow passage 12 is increased, and thus the displacement amount of the slide sleeve 24 and the deformation amount of the elastic body 23 are both increased, and the inner diameter of the portion of the water discharge hose corresponding to the elastic body 23 is reduced, and the water discharge velocity becomes faster, that is, as the suction power is increased, the water discharge velocity is also increased, therefore, the problem that the water from the water discharge pipe is drawn away before it reaches the skin when the suction power is increased can be avoided. When the suction power is reduced, the pressure in the reflow passage 12 is reduced, thus the displacement amount of the slide sleeve 24 and the deformation amount of the elastic body 23 are also reduced, the inner diameter of the portion of the water discharge hose corresponding to the elastic body 23 is enlarged, and the water discharge velocity of the cleaning head is slowed down accordingly, that is, as the suction power is reduced, the water discharge velocity of the cleaning head is also slowed down, therefore, the problem that the dirty water cannot be drawn away after cleaning the skin in time can be avoided. After the cleaning is completed, when the cleaning head is moved away from the skin, the air pressure in the reflow passage 12 is recovered to be equal to the atmospheric pressure, and thus the water will not be sprayed out any more. Therefore, as long as water discharging and air suction are performed at the same time, the body cleaning tool can also automatically adjust the open or close of the water discharge hose 22 according to whether or not the cleaning head contacts with the skin.

The slide sleeve 24 may be a cylindrical sleeve or a conical sleeve; where a cylindrical sleeve is used, the area to be forced is the bottom surface of the cylinder, which therefore facilitates the slide sleeve 24 to be pushed by force to move. In one embodiment of the present disclosure, the slide sleeve 24 is a conical sleeve, and the end with a smaller diameter is provided near the suction port 11 of the outer case 1, and the pressure in the reflow passage 12 is acted on the end with a smaller diameter so that both this end and the conical side surface are forced, the area to be acted is increased, thus the slide sleeve 24 can be more easily moved under the effect of the pressure.

In order that the elastic body 23 is more stably operated, an annular slot 241 is provided in the inner wall of the slide sleeve 24, and the elastic body 23 is provided in the annular slot 241, and an end of the pipe bracket 21 is inserted into the annular slot 241 and is abutted against the elastic body 23, so that the elastic body 23 is firmly held in the annular slot 241, and the reliability of the elastic body 23 upon operation is guaranteed. In one embodiment of the present disclosure, the elastic body 23 may be made from a rubber material.

The elastic body 23 may include a plurality of elastic blocks distributed in the annular slot 241, the elastic block each may be of a sphere shape or a cubic shape. The elastic body 23 may also be a single elastic ring. In one embodiment, to guarantee the operation stability of the elastic body 23, when the elastic body 23 is of a elastic ring, in order to avoid the response delay when the elastic ring is pressed, the maximum diameter of the annular slot 241 is comparable with the outer diameter of the elastic ring, once the slide sleeve 24 is displaced, a deformation amount is occurred immediately in the elastic ring and acts on the water discharge hose 22, thus it can be guaranteed that the water discharge velocity can be varied upon the suction power being changed, and thus the cleaning effect can be guaranteed.

In order to guarantee sufficient time for the water sprayed from the water outlet of the water discharge hose 22 to clean the skin before it is drawn away, the water outlet of the water discharge hose 22 should be positioned within the outer case 1, and the suction port 11 of the outer case 1 is provided at a distance apart from the water outlet of the water discharge hose 22 along the axial direction.

In one embodiment of the present disclosure, the distance by which the suction port 11 of the outer case 1 is separated away from the water outlet of the water discharge hose 22 along the axial direction should be less than or equal to a spray distance of the water outlet; with this configuration, upon operation, the water can be spray onto the skin at a suitable force.

In order to avoid the problem that the water from the water outlet brings uncomfortable feeling to the skin due to the spray distance is too large or the water velocity is too fast in cleaning the skin, the difference between the distance by which the suction port 11 of the outer case 1 is separated away from the water outlet of the water discharge hose 22 along the axial direction and the spray distance of the water outlet should not be too large, for example, the difference is from 1 cm to 2 cm.

At least one embodiment of the present disclosure also provides a body cleaning tool, the body cleaning tool includes a water supply motor (not shown in figures) and a suction motor (not shown in figures), and further includes a body cleaning head as described in any of the above embodiments, the water supply motor communicates with the water discharge hose 22, and the suction motor communicates with the reflow passage 12 of the body cleaning head, the ambient air and the water sprayed out of the water discharge hose 22 can be drawn back into the cleaning tool by the suction motor through the reflow passage 12.

By the restriction of the two conditions of the constant water pressure and the limited deformation amount, the water outflow velocity is limited in a certain range; therefore, the amount of the corresponding suction force should also be limited in a certain range. If the suction force is too large, the water discharge hose 22 will always be closed; and if the suction force is too small, the water outflow velocity is excessively fast, and will bring uncomfortable feeling to the user upon cleaning, and the sprayed water may not be fully recovered. Therefore, in selecting the elastic body 23, the elastic coefficient of the elastic body 23 should match with the power of the suction motor and the power of the water supply motor.

Other components for the body cleaning tool of the embodiments of the present disclosure may adopt those well known by the person skilled in the art and will not be further described in detail.

The features, structures, materials or the characters described in the present disclosure can be combined in any suitable way in any one or more embodiments or examples.

The present disclosure has been described above by way of the exemplary embodiment, and the protection scope of the present invention would not be limited therein, and is only defined by the following claims.

The present application claims the priority of Chinese Patent Application No. 201410486475.6 filed on September 22, 2014.

## Claims

1. A body cleaning head comprising:
an outer case (1) provided with a suction port (11) and provided therein with a reflow passage (12) communicating with the suction port (11); and
a water pipe assembly (2) provided within the reflow passage (12), wherein the water pipe assembly (2) includes a pipe bracket (21) fixedly provided in the outer case (1), a water discharge hose (22) is inserted and fitted within the pipe bracket (21) and an end of the water discharge hose (22) adjacent to the suction port (11) is protruded from the pipe bracket (21);
the body cleaning head being **characterized in that**, an elastic body (23) is provided to sleeve the end of the water discharge hose (22) at the end of the pipe bracket (21) and a slide sleeve (24) is provided to sleeve the elastic body (23) and the water discharge hose (22), both the elastic body (23) and the slide sleeve (24) are configured to surround the water discharge hose (22), the elastic body (23) is located between the pipe bracket (21) and the slide sleeve (24), when the body cleaning head sucks air, the slide sleeve (24) axially moves relative to the pipe bracket (21) under an action of the airflow pressure in such a manner that the elastic body (23) is deformed such that an inner diameter of the elastic body (23) is reduced and accordingly, an inner diameter of a portion of the water discharge hose (22) corresponding to the elastic body (23) is varied with the airflow pressure.

2. The body cleaning head according to claim 1, wherein the slide sleeve (24) is a conical sleeve, and an end of the slide sleeve (24) with a smaller diameter is provided adjacent to the suction port (11) of the outer case (1).

3. The body cleaning head according to claim 1 or claim 2, wherein, an annular slot (241) is formed in the inner wall of the slide sleeve (24), the elastic body (23) is placed in the annular slot (241), and an end of the pipe bracket (21) is inserted into the annular slot (241) and abuts against the elastic body (23).

4. The body cleaning head according to claim 3, wherein the elastic body (23) is an elastic ring, and the maximum diameter of the annular slot (241) is comparable with the outer diameter of the elastic ring.

5. The body cleaning head according to any one of claims 1 to 3, wherein the elastic body (23) includes a plurality of elastic blocks.

6. The body cleaning head according to any one of claims 1 to 5, wherein a water outlet of the water discharge hose (22) is located in the outer case (1), and the suction port (11) of the outer case (1) is provided at a distance away from the water outlet of the water discharge hose (22) along an axial direction.

7. The body cleaning head according to any one of claims 1 to 6, wherein the distance by which the suction port (11) of the outer case (1) is separated from the water outlet of the water discharge hose (22) along the axial direction is less than or equal to a spray distance of the water outlet.

8. The body cleaning head according to any one of claims 1 to 7, wherein the distance by which the suction port (11) of the outer case (1) is separated from the water outlet of the water discharge hose (22) along the axial direction is different from the spray distance by 1 to 2 cm.

9. The body cleaning head according to any one of claims 1 to 8, wherein the elastic body (23) is made from a rubber material.

10. A body cleaning tool comprising a water supply motor and a suction motor, and further comprising a body cleaning head according to any one of claims 1 to 9, wherein the water supply motor communicates with the water discharge hose (22), the suction motor communicates with the reflow passage (12) of the body cleaning head, ambient air and water flow sprayed from the water discharge hose (22) can be drawn back into the cleaning tool by the suction motor through the reflow passage (12).

## Patentansprüche

1. Körperreinigungskopf, umfassend:
ein äußeres Gehäuse (1), versehen mit einem Saugmund (11) und darin versehen mit einer Rückflusspassage (12), in Verbindung stehend mit dem Saugmund (11); und
eine Wasserleitungsanordnung (2), bereitgestellt innerhalb der Rückflusspassage (12), wobei die Wasserleitungsanordnung (2) einen Leitungshalter (21), der in dem äußeren Gehäuse (1) fest vorgesehen ist, beinhaltet, wobei ein Wasserförderschlauch (22) innerhalb des Leitungshalters (21) befestigt ist und wobei ein Ende des Wasserförderschlauchs (22) benachbart zu dem Saugmund (11) von dem Leitungshalter (21) hervorsteht;
wobei der Körperreinigungskopf **dadurch gekennzeichnet ist, dass** ein elastischer Körper (23) vorgesehen ist, um das Ende des Wasserförderschlauchs (22) an dem Ende des Leitungshalters (21) zu umhüllen, und wobei eine Gleithülse (24) vorgesehen ist, um den elastischen Körper (23) und den Wasserförderschlauch (22) zu umhüllen, wobei sowohl der elastische Körper (23) als auch die Gleithülse (24) konfiguriert sind, um den Wasserförderschlauch (22) zu umgeben, wobei der elastische Körper (23) zwischen dem Leitungshalter (21) und der Gleithülse (24) lokalisiert ist, und wobei, wenn der Körperreinigungskopf Luft saugt, die Gleithülse (24) sich axial relativ zu dem Leitungshalter (21) bewegt unter einer Wirkung des Luftstromdrucks in einer solchen Weise, dass der elastische Körper (23) verformt wird, so dass ein innerer Durchmesser des elastischen Körpers (23) verringert wird und demgemäß ein innerer Durchmesser eines Bereiches des Wasserförderschlauchs (22) korrespondierend zu dem elastischen Körper (23) mit dem Luftstromdruck variiert wird.

2. Körperreinigungskopf gemäß Anspruch 1, wobei die Gleithülse (24) eine konische Hülse ist und wobei ein Ende der Gleithülse (24) mit einem kleineren Durchmesser benachbart zu dem Saugmund (11) des äußeren Gehäuses (1) vorgesehen ist.

3. Körperreinigungskopf gemäß Anspruch 1 oder Anspruch 2, wobei ein ringförmiger Schlitz (241) in der inneren Wand der Gleithülse (24) gebildet ist, wobei der elastische Körper (23) in dem ringförmigen Schlitz (241) platziert ist und wobei ein Ende des Leitungshalters (21) in den ringförmigen Schlitz (241) eingesetzt ist und gegen den elastischen Körper (23) angrenzt.

4. Körperreinigungskopf gemäß Anspruch 3, wobei der elastische Körper (23) ein elastischer Ring ist und wobei der maximale Durchmesser des ringförmigen Schlitzes (241) vergleichbar mit dem äußeren Durchmesser des elastischen Rings ist.

5. Körperreinigungskopf gemäß einem der Ansprüche 1 bis 3, wobei der elastische Körper (23) eine Mehrzahl von elastischen Blöcken beinhaltet.

6. Körperreinigungskopf gemäß einem der Ansprüche 1 bis 5, wobei ein Wasserauslass des Wasserförderschlauchs (22) in dem äußeren Gehäuse (1) lokalisiert ist, und wobei der Saugmund (11) des äußeren Gehäuses (1) entlang einer axialen Richtung in einem Abstand entfernt von dem Wasserauslass des Wasserförderschlauchs (22) vorgesehen ist.

7. Körperreinigungskopf gemäß einem der Ansprüche 1 bis 6, wobei der Abstand, mittels welchem der Saugmund (11) des äußeren Gehäuses (1) entlang der axialen Richtung von dem Wasserauslass des Wasserförderschlauchs (22) separiert ist, kleiner als eine oder gleich zu einer Spray-Distanz des Wasserauslasses ist.

8. Körperreinigungskopf gemäß einem der Ansprüche 1 bis 7, wobei der Abstand, mittels welchem der Saugmund (11) des äußeren Gehäuses (1) entlang der axialen Richtung von dem Wasserauslass des Wasserförderschlauchs (22) separiert ist, um 1 bis 2 cm verschieden von der Spray-Distanz ist.

9. Körperreinigungskopf gemäß einem der Ansprüche 1 bis 8, wobei der elastische Körper (23) aus Gummimaterial hergestellt ist.

10. Körperreinigungswerkzeug, umfassend einen Wasserzufuhrmotor und einen Saugmotor, und außerdem umfassend einen Körperreinigungskopf gemäß einem der Ansprüche 1 bis 9, wobei der Wasserzufuhrmotor mit dem Wasserförderschlauch (22) in Verbindung steht, wobei der Saugmotor mit der Rückflusspassage (12) des Körperreinigungskopfs in Verbindung steht, wobei Umgebungsluft und Wasserfluss, gesprüht von dem Wasserförderschlauch (22), mittels des Saugmotors durch die Rückflusspassage (12) in das Reinigungswerkzeug zurückgezogen werden können.

## Revendications

1. Une tête de nettoyage du corps comprenant :
un boîtier extérieur (1) muni d'un orifice/ouverture d'aspiration (11) et muni d'un passage de refoulement (12) communiquant avec l'orifice/ouverture d'aspiration (11) ; et
un assemblage de conduite d'eau (2) prévu dans le passage de refoulement (12), dans lequel l'assemblage de conduite d'eau (2) comprend un support de conduite (21) prévu de manière fixe dans le boîtier extérieur (1), un tuyau d'évacuation d'eau (22) est inséré et monté dans le support de conduite (21) et une extrémité du tuyau d'évacuation d'eau (22) adjacente à l'orifice/ouverture d'aspiration (11) dépasse du support de conduite (21) ;
la tête de nettoyage du corps étant **caractérisée en ce qu'**un corps élastique (23) est prévu pour entourer l'extrémité du tuyau d'évacuation d'eau (22) à l'extrémité du support de conduite (21) et un manchon coulissant (24) est prévu pour entourer le corps élastique (23) et le tuyau d'évacuation d'eau (22), le corps élastique (23) et le manchon coulissant (24) sont tous deux configurés pour entourer le tuyau d'évacuation d'eau (22), le corps élastique (23) est placé entre le support de tuyau (21) et le manchon coulissant (24), lorsque la tête de nettoyage du corps aspire de l'air, le manchon coulissant (24) se déplace axialement par rapport au support de tube (21) sous une action de la pression du flux d'air de telle manière que le corps élastique (23) soit déformé de manière qu'un diamètre intérieur du corps élastique (23) soit réduit et, en conséquence, un diamètre intérieur d'une partie du tuyau de décharge (22) correspondant au corps élastique (23) est modifié avec la pression du flux d'air.

2. La tête de nettoyage du corps selon la revendication 1, dans laquelle le manchon coulissant (24) est un manchon conique, et une extrémité du manchon coulissant (24) d'un diamètre inférieur est prévue adjacente à l'orifice/ouverture d'aspiration (11) du boîtier extérieur (1).

3. La tête de nettoyage du corps selon la revendication 1 ou la revendication 2, dans laquelle une fente annulaire (241) est formée dans la paroi intérieure du manchon coulissant (24), le corps élastique (23) est placé dans la fente annulaire (241) et une extrémité du support de tuyau (21) est introduite dans la fente annulaire (241) et vient en appui contre le corps élastique (23) .

4. La tête de nettoyage du corps selon la revendication 3, dans laquelle le corps élastique (23) est un anneau élastique et le diamètre maximal de la fente annulaire (241) est comparable au diamètre extérieur de l'anneau élastique.

5. La tête de nettoyage du corps selon l'une quelconque des revendications 1 à 3, dans laquelle le corps élastique (23) comprend une pluralité de blocs élastiques.

6. La tête de nettoyage du corps selon l'une quelconque des revendications 1 à 5, dans laquelle une sortie d'eau du tuyau d'évacuation d'eau (22) est située dans le boîtier extérieur (1) et l'orifice/ouverture d'aspiration (11) du boîtier extérieur (1) est prévu à distance de la sortie d'eau du tuyau d'évacuation d'eau (22) suivant une direction axiale.

7. La tête de nettoyage du corps selon l'une quelconque des revendications 1 à 6, dans laquelle la distance par laquelle l'orifice/ouverture d'aspiration (11) du boîtier extérieur (1) est séparé de la sortie d'eau du tuyau d'évacuation d'eau (22) dans la direction axiale est inférieure ou égale à une distance de pulvérisation de la sortie d'eau.

8. La tête de nettoyage du corps selon l'une quelconque des revendications 1 à 7, dans laquelle la distance par laquelle l'orifice/ouverture d'aspiration (11) du boîtier extérieur (1) est séparé de la sortie d'eau du tuyau d'évacuation d'eau (22) dans la direction axiale est différente de 1 à 2 cm de la distance de pulvérisation.

9. La tête de nettoyage du corps selon l'une quelconque des revendications 1 à 8, dans laquelle le corps élastique (23) est réalisé en un matériau en caoutchouc.

10. Un outil de nettoyage du corps comprenant un moteur d'alimentation en eau et un moteur d'aspiration, et comprenant en outre une tête de nettoyage du corps selon l'une quelconque des revendications 1 à 9, dans lequel le moteur d'alimentation en eau communique avec le tuyau d'évacuation d'eau (22), le moteur d'aspiration communique avec le passage de refoulement (12) de la tête de nettoyage du corps, l'air ambiant et l'eau pulvérisés par le tuyau de refoulement d'eau (22) peuvent être renvoyés par le moteur d'aspiration dans l'outil de nettoyage à travers le passage de refoulement (12).
